# EUROPEAN PATENT SPECIFICATION

(11) **EP 3 228 348 B1**
(45) Date of publication and mention of the grant of the patent: **05.06.2019**
(21) Application number: 17165029.4
(22) Date of filing: 05.04.2017
(51) Int. Cl.: A61M 25/01, A61M 25/10, A61B 8/12, A61L 29/14, A61K 49/22

(54) **MEDICAL DEVICE IMAGABLE BY ULTRASOUND**
MITTELS ULTRASCHALL DARSTELLBARE MEDIZINISCHE VORRICHTUNG
DISPOSITIF MÉDICAL D'IMAGERIE PAR ULTRASONS

(30) Priority: 06.04.2016 US 201615092023
(43) Date of publication of application: 11.10.2017
(73) Proprietor: Covidien LP, Mansfield, MA 02048 (US)
(72) Inventor: McPeak, Thomas, Santa Rosa, CA California 95403 (US)
(74) Representative: Zimmermann & Partner Patentanwälte mbB

(56) References cited:
- EP-A1- 1 462 056
- WO-A1-00/51136
- WO-A1-94/21175
- WO-A1-98/19713

## Description

### FIELD

The present disclosure relates to, among other things, medical devices, or portions thereof, visible via ultrasound.

### TECHNICAL BACKGROUND

Many medical procedures, whether interventional or diagnostic, include use of instruments that are, or have markers that are, visible by fluoroscopy. Fluoroscopy requires the use of radiation for visualization, which may present health concerns for both patients and clinicians.

WO 00/51136 A1 relates to medical tools and devices with improved ultrasound visibility.

WO 98/19713 A1 relates to an echogenic coating containing gaseous spaces for ultrasonography.

EP 1 462 056 A1, according to its abstract, relates to an embryo transfer catheter that has a shaft extruded with two layers, wherein the outer layer is relatively thick and contains gas bubbles sufficient to increase the visibility of the catheter under ultrasound observation but with a density that allows material within the catheter to be viewed by the eye.

Ultrasound techniques, which do not employ radiation, provide an alternative to fluoroscopy. However, many medical devices do not provide for sufficient contrast for accurate imaging via ultrasound.

### BRIEF SUMMARY

Described herein, among other things, are medical devices that are configured to provide good contrast for viewing by ultrasound. The devices include material that contains or releases gas to enhance ultrasound echogenicity. In some embodiments, the devices, or one or more portions thereof, are coated with a material that releases gas, such as carbon dioxide, when introduced into a patient. In many embodiments, the devices are configured for placement or movement through a patient's vasculature. As the device is moved through the patient, the location of the device or one or more portion thereof may be effectively monitored by ultrasound.

In general, in one aspect, the present disclosure describes a medical device that includes a structural element comprising an outer surface and a material on the outer surface of the structural element. The material defines void spaces containing a gas entrained within the void spaces. The material, upon contact with physiological fluid, is configured to release the gas. The material is configured to release the gas in an amount sufficient for the gas to be imaged by extracorporeal ultrasound.

In various embodiments, the device further comprises a coating on the outer surface of the device, wherein the coating comprises the material. In certain embodiments, the coating comprises a hydrogel.

The material dissolves in physiological fluid. In various embodiments, the material has a solubility of 100 mg/ml or more in pH 7.4 phosphate buffered saline at room temperature.

In various embodiments, the material defining the void space in which the gas is entrained comprises a monosaccharide or polysaccharide. In certain embodiments, the monosaccharide or polysaccharide comprises sucrose or glucose.

In embodiments, the device further comprises an outer coating disposed over the material defining the void space in which the gas is entrained. In certain embodiments, the outer coating is a hydrophilic lubricous coating.

In embodiments, the material defining the void space in which the gas is entrained comprises a plurality of microspheres, each defining an interior volume, and the gas is present within that interior volume. In various embodiments, a liquid is in the interior volume of the microspheres and the gas is entrained in the liquid. In various embodiments, the gas comprises carbon dioxide.

In embodiments, the structural element comprises an inflatable balloon. In other embodiments, the structural element comprises a catheter. In various embodiments, the structure comprises an inflatable balloon and a catheter.

In general, in another aspect, the present disclosure describes a method comprising providing a medical device of any of the embodiments described above, or combinations thereof. The structural element of the medical device may be inserted into a patient's vasculature, and the location of the structural element within the vasculature may be identified via extravascular ultrasound.

In embodiments, the structural element is an inflatable balloon, and the method may further comprise inflating the inflatable balloon within the vasculature. The method may further comprise imaging inflation of the inflatable balloon via extravascular ultrasound.

In general, in yet another aspect, the present disclosure describes a method of making a medical device. A gas is entrained into a material, wherein the material defines void spaces and the gas is entrained within the void spaces. The material is disposed on an outer surface of a structural element of the medical device. The material is configured to release the gas from the voids in the material upon contact with a physiological fluid in an amount sufficient for the released gas to be imaged by extracorporeal ultrasound.

In embodiments, the structural element is an inflatable balloon. In various embodiments, the gas is carbon dioxide. In various embodiments, the material may be at least one of a water-soluble polymer, a monosaccharide, a polysaccharide, and microspheres.

The disclosure can be implemented to realize one or more of the following advantages. The medical device can be visualized using ultrasound, thereby eliminating the need for fluoroscopy, and thus not subjecting the patient to radiation. Further, the release of gas may also help the physician also image the vessel in which the medical device is being used. Further still, more of the medical device and structure may be imaged as compared to the use of narrow, discrete marker bands. Additional advantages and features of the subject matter of the present disclosure will be set forth in the detailed description which follows, and in part will be readily apparent to those skilled in the art from that description or recognized by practicing the subject matter of the present disclosure as described herein, including the detailed description which follows, the claims, as well as the appended drawings.

It is to be understood that both the foregoing general description and the following detailed description present embodiments of the subject matter of the present disclosure, and are intended to provide an overview or framework for understanding the nature and character of the subject matter of the present disclosure as it is claimed. The accompanying drawings are included to provide a further understanding of the subject matter of the present disclosure, and are incorporated into and constitute a part of this specification. The drawings illustrate various embodiments of the subject matter of the present disclosure and together with the description serve to explain the principles and operations of the subject matter of the present disclosure. Additionally, the drawings and descriptions are meant to be merely illustrative, and are not intended to limit the scope of the claims in any manner.

### BRIEF DESCRIPTION OF THE DRAWINGS

The following detailed description of specific embodiments of the present disclosure can be best understood when read in conjunction with the following drawings, in which:
**FIG. 1** is a schematic sectional view of an embodiment of a medical device having a gas-containing material on an exterior surface of a structural element;
**FIG. 2** is a schematic sectional view of an embodiment of a medical device having a gas-containing material on an exterior surface of a structural element and a coating on the gas-containing material;
**FIG. 3** is a schematic sectional view of an embodiment of a medical device having a coating on an exterior surface of a structural element where the coating includes a gas-containing material; and
**FIG. 4** is a schematic sectional view of a balloon catheter in accordance with various embodiments described herein.

The schematic drawings are not necessarily to scale.

### DETAILED DESCRIPTION

Reference will now be made in greater detail to various embodiments of the subject matter of the present disclosure, some embodiments of which are illustrated in the accompanying drawings. Like numbers used in the figures refer to like components, steps and the like. However, it will be understood that the use of a number to refer to a component in a given figure is not intended to limit the component in another figure labeled with the same number. In addition, the use of different numbers to refer to components in different figures is not intended to indicate that the different numbered components cannot be the same or similar to other numbered components.

The present disclosure describes, among other things, a medical device including a structural element comprising an outer surface and a material on the outer surface of the structural element. The material defines void spaces and contains a gas entrained within the void spaces. The material, upon contact with physiological fluid, is configured to release the gas in an amount sufficient for the gas to be imaged by ultrasound.

Any suitable medical device may include a gas-containing material as described herein. Any device having at least a portion that is inserted into a patient may advantageously include a gas-containing material as described herein. In some embodiments, the device is configured to be placed in or moved through a vasculature of a patient. For example, the device may be an angioplasty device, a stent delivery device, or a diagnostic device. In some embodiments, the device is an implantable medical device; e.g., a device configured to remain in a patient for more than a week, such as a stent.

The material may be located at any suitable portion of the device or over the entire device. Preferably the material is positioned at an external surface of the device that is configured to contact physiological fluid of a patient. In some embodiments, the material is at locations in which radiopaque material or marker bands are employed for currently available devices. For example, if the material is present on a stent delivery catheter, the material may form one or more bands distal to, proximal to, or at a location where the stent is configured to be retained. However, an advantage of using this material as a coating is the ability to visualize more of the device than just defined bands.

In some embodiments, the device comprises a balloon catheter and the balloon comprises a coating having the gas-releasing material, or a sheet of the gas releasing material is disposed about the balloon. Accordingly, the balloon may be effectively visualized by ultrasound as the balloon is moved through the vasculature. In some embodiments, such as when the balloon is coated with the gas-releasing material, inflation of the balloon in the vasculature may be visualized by ultrasound, which can provide feedback to a clinician of effectiveness of treatment.

Any suitable material may be used to entrain gas to be released. The material defines void spaces in which the gas is entrained. The material is configured to release the gas when in contact with physiological fluid, such as blood or interstitial fluid. The material may dissolve, degrade, invert, chemically react, or otherwise change state or orientation to release the entrained gas when placed in contact with physiological fluid. The release of the gas preferably occurs over time, particularly over the time that a particular procedure employing a device including such a material may take.

Examples of suitable materials in which the gas may be entrained include sugars, water soluble biocompatible polymers, and microspheres. Suitable sugars may be monosaccharides or polysaccharides, such as disaccharides. Suitable sugars include glucose, fructose, sucrose, xylose and the like. The sugars may be heated until they melt into a syrup. The syrup may be exposed to pressurized gas and allowed to cool and solidify causing high-pressure gas to be trapped inside the resultant solid. The syrup can be exposed to any suitable pressure of gas. For example, the pressure may be 0.5 bar or greater, 1 bar or greater, 5 bar or greater, 10 bar or greater, or 20 bar or greater. Typically the pressure of the gas will be less than 100 bar. In some embodiments, the pressure of the gas will be from about 20 bar to about 60 bar, such as from about 30 bar to about 50 bar, or about 40 bar. The resulting solid may be molded into a desired shape to be placed about a medical device, broken into pieces of suitable size for incorporating into a coating to be placed on a medical device, or the like, or a medical device or portion thereof may be molded around at least a portion of the resulting solid. For example, a ring of the resulting solid may be placed in a mold in which a catheter is formed so that the ring can form an ultrasonic marker band of gas-containing material.

Any suitable water soluble biocompatible polymer may be used to entrain gas. Preferably, the material includes a thermoplastic water soluble polymer. Examples of thermoplastic water soluble polymers include polyethylene oxide (such as POLYOX water-soluble poly(ethylene) oxide polymers available from the Dow Chemical Company), polyvinyl alcohol, and cellulose ethers, such as hydroxypropyl methylcellulose and hydroxyethyl methylcellulose. One or more water soluble thermoplastic polymers may be used to form a material in which gas may be entrained. In some embodiments, the one or more water soluble thermoplastic polymers are heated until they melt, exposed to pressurized gas, and cooled to form a solid in which high-pressure gas may be trapped. The melt can be exposed to any suitable pressure of gas. For example, the pressure may be 0.5 bar or greater, 1 bar or greater, 5 bar or greater, 10 bar or greater, or 20 bar or greater. Typically the pressure of the gas will be less than 100 bar. In some embodiments, the pressure of the gas will be from about 20 bar to about 60 bar, such as from about 30 bar to about 50 bar, or about 40 bar. The resulting solid may be molded into a desired shape to be placed about a medical device, a medical device or portion thereof may be molded around at least a portion of the resulting solid, or the like. For example, a ring of the resulting solid may be placed in a mold in which a catheter is formed so that the ring can form an ultrasonic marker band of gas-containing material.

In some embodiments, the material in which the gas is entrained has a solubility in physiological saline at pH 7.4 and 37°C of 50 mg/ml or more, such as 100 mg/ml or more. In some embodiments, the material will have a solubility in physiological saline at pH 7.4 and 37°C of less than 5000 mg/ml.

Further, any suitable microspheres may be used to entrain gas. For example, the microsphere may include an amphiphilic polymer, such as a block copolymer having one or more hydrophobic blocks and one or more hydrophilic blocks. Upon contact with physiological fluid, the microspheres may release the gas contained within the microsphere. The gas may be, for example, in a super-saturated solution within the microsphere.

Examples of select microspheres known in the art that may be used to contain a gas include, but are not limited to, poly(D,L- lactide-co-glycolide) (PLGA) microspheres; poly(epsilon-caprolactone) (PCL) microspheres; poly(D,L-lactide)/poly(D,L-lactide-co-glycolide) composite microparticles; alginate-poly-L- lysine alginate (APA) microcapsules; alginate microspheres; poly(D,L-lactic-co-glycolic acid) microspheres; chitosan microspheres; poly[p-(carboxyethylformamido)-benzoic anhydride] (PCEFB) microspheres; Hyaluronan-based microspheres; biodegradable microspheres; microspheres of PMMA-PCL-cholesterol; poly(propylene fumarate)/poly(lactic-co-glycolic acid) blend microspheres; poly(lactide-co-glycolide acid-glucose) microspheres; polylactide co-glycolide (PLG) microspheres; poly(methacrylic acid) microspheres; poly(methylidene malonate 2.1.2)-based microspheres; ammonio methacrylate copolymer microspheres; poly(ethylene oxide)-modified poly(beta-amino ester) microspheres; methoxy poly(ethylene glycol)/poly(epsilon-caprolactone) microspheres; polyferrocenylsilane microspheres; poly(fumaric-co-sebacic acid) (P(FASA)) microspheres; poly(lactide-co-glycolide) and poly(ethylene oxide)-poly(propylene oxide)-poly(ethylene oxide) triblock copolymer microspheres; polyisobutylcyanoacrylate microspheres; polystyrene core-glycopolymer corona nanospheres; poly(ethyl-2-cyanoacrylate) microspheres; POE-PEG-POE triblock copolymeric microspheres; poly(DL-lactide) microparticles; albumin microspheres; poly(EGDMA/HEMA) based microbeads; glutaraldehyde crosslinked sodium alginate microbeads; pectin microspheres; methoxy poly(ethylene glycol) and glycolide copolymer microspheres; crosslinked polyethyleneimine microspheres; poly(glycidyl methacrylate-co-ethylene dimethacrylate); cellulose acetate trimellitate ethylcellulose blend microspheres; poly(ester) microspheres; polyacrylamide microcarriers; polyacrolein microspheres; 2- hydroxyethyl methacryiate microspheres

The microspheres may be of varying size. Suitable size microspheres include those ranging from between about 10 and about 5000 nm in outside diameter, preferably between about 50 and about 500 nm in outside diameter. Most preferably, the microspheres are about 75 nm to about 200 nm in outside diameter.

The microspheres may be prepared by various processes, as will be readily apparent to those skilled in the art, such as by interfacial polymerization, phase separation and coacervation, multiorifice centrifugal preparation, and solvent evaporation, or a combination thereof. The microspheres may be formed under pressurized gas so that the gas may be captured in or entrained within liquid in the microsphere. Any suitable pressure may be employed. For example, the pressure may be 0.5 bar or greater, 1 bar or greater, 5 bar or greater, 10 bar or greater, or 20 bar or greater. Typically the pressure of the gas will be less than 100 bar. In some embodiments, the pressure of the gas will be from about 20 bar to about 60 bar, such as from about 30 bar to about 50 bar, or about 40 bar.

Microspheres or other suitable gas-containing material may be included in a coating applied to a medical device, may be applied to the device and overcoated with an outer coating, or the like. The coating or outer coating may assist in retaining the gas-containing material in place on the device, may serve to facilitate movement of the device through the patient, or the like. Preferably, the coating or outer coating allows the material to come in contact with body fluid. Alternatively or in addition, the coating or outer coating may react with body fluid to cause the material to release the gas.

In some embodiments, the coating or outer coating is a lubricious hydrophilic coating. Any suitable lubricious coating may be employed. Preferably, the hydrophilic nature of lubricious coatings allows physiological fluid to reach the gas-containing material to cause release of the gas from the material when the device is inserted in a patient. The references to coatings in general below refer to both the coating in which the gas may be entrained and potential outer coatings.

Examples of suitable lubricious coatings include coatings comprising one or more of polyvinylpyrrolidone, polyethylene oxide, hyaluronic acid and its salts, polyacrylic acid and its derivatives, polyurethane, acrylic polyester, and the like.

In some embodiments, the coating comprises a polysaccharide. The polysaccharide may be cross-linked. For example, the polysaccharide may be chemically crosslinked glutaraldehyde or another compound having at least two aldehyde groups. By way of another example, crosslinking may be ionic. Ionic cross-linking may be particularly suitable for polysaccharides having acid functional groups, such as glycosaminoglycans. Examples of suitable glycosaminoglycans having acid functional groups include hyaluronic acid, xanthan gum, carrageenan, tragacanth, gellan gum and pectins. Crosslinking may be provided with a biocompatible polyvalent cation such as calcium, magnesium or iron. In some cases a polymer with multiple cationic groups may be utilized.

In some embodiments, the coating comprises hyaluronic acid or a salt thereof. Hyaluronic Acid (HA) is a water soluble high molecular weight (MW) polysaccharide commonly found in normal human tissue, as well as a number of other natural sources. It is biocompatible, non-thrombogenic, highly hydrophilic, hydroscopic, and with a high intrinsic viscosity at high MW and concentration. HA is highly insoluble in most organic solvents and will remain stable during most drug coating processes that include organic solvents. HA can be of various MW, although MWs of greater than or equal to 1x10⁶ Da are preferred due to a higher degree of chain interaction. In some embodiments the coating consists of or consists essentially of HA. In some embodiments, the coating may include HA mixed with varying amounts of another polymer, such as PVA or collagen, to adjust visco-elastic properties. In some embodiments, the HA is cross-linked to increase stability of the resulting HA layer or alter the rate of degradation.

The material or the material and the coating, if present, may be adjusted to tailor the release profile of the gas from the material following insertion of the device in the patient. Preferably, the material releases gas over the course of a procedure in which the medical device is employed or during a procedure in which the medical device is implanted. Preferably, gas is released from the material on the medical device within 5 minutes of contact with physiological fluid; more preferably within 1 minute, and even more preferably within seconds of contact with physiological fluid. Preferably, gas is released from the material on the medical device until the device is removed from the patient or implanted in the patient. For example and in some embodiments, gas may be released for up to 1 hour when material on the device is in contact with physiological fluid. In some embodiments, release for up to 30 minutes, up to 15 minutes, up to 10 minutes, up to 5 minutes, or even up to one minute will be sufficient.

It will be understood that the gas release profile and the location of the gas-containing material on the device may vary depending of the medical device and the procedure for which the device is employed.

The gas-containing material may entrain any suitable gas within a void space defined by the material. For example, the gas may comprise one or more of oxygen, carbon dioxide, and nitrogen. Carbon dioxide should not have therapeutic or side effects, while oxygen and nitrogen may have desired therapeutic effects or unintended side effects. It will be understood that the choice of gas employed will depend, in part, on the medical device, the procedure for which the device is employed, the location of the gas-containing material on the device, and the like.

Preferably, the gas is present in the material at a pressure above atmospheric pressure. More preferably, the gas is present in the material at a pressure above a relevant physiological pressure, such as blood pressure which is about 0.2 bar. Preferably, the gas is present in the material at a pressure below a pressure that may result in a release rate that is too high for patient safety. The maximum pressure for a gas in a material may depend on, not only the pressure, but also the material in which the gas is entrained, the area of the body in which a device containing the material is used, and the like. In some embodiments, the gas entrained in a void space of the material may be at a pressure of from about 0.2 bar to about 50 bar such as from about 1 to about 40, or about 2 bar to about 20 bar. For purposes of the present disclosure, pressures referred to herein are gauge pressures.

Preferably, gas bubbles are formed in a physiological fluid when the gas is released from the material in the fluid. Preferably, a sufficient amount of gas bubbles are formed to enhance ultrasound echogenicity. Preferably, the gas is released from the material as gas bubbles having an outer diameter in a range from about 1 micrometer to about 1000 micrometers. Bubbles of such sizes should be sufficient to enable effective visualization by ultrasound, such as extracorporeal ultrasound.

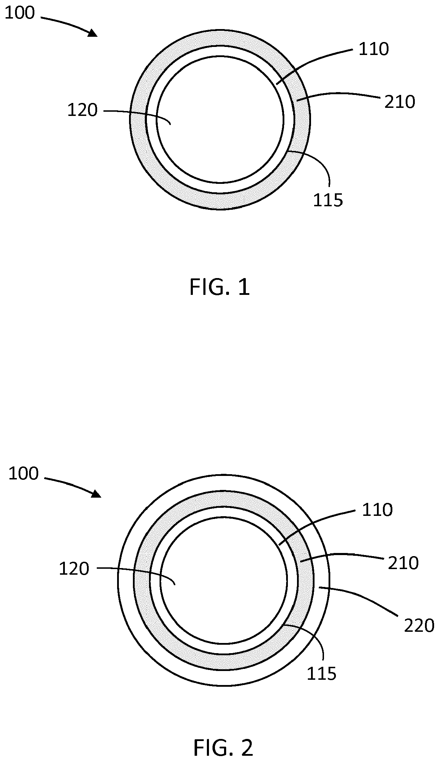

Referring now to **FIG. 1****,** a sectional view of a medical device **100** is shown. The device **100** includes a structural element **110** having an external surface **115,** and a gas-containing material **210** on the external surface **115** of the structural element **110.** In the depicted embodiment, the structural element **110** forms a lumen **120.** For example, the structural element may comprise a catheter or an inflatable balloon.

Referring now to **FIG. 2****,** a sectional view of a medical device **100** is shown. The device **100** includes a structural element **110,** a gas-containing material **210** on an external surface **115** of the structural element **110,** and an outer coating **220** on the material **210.** The material **210** may be coated or otherwise disposed on the structural element **110.**

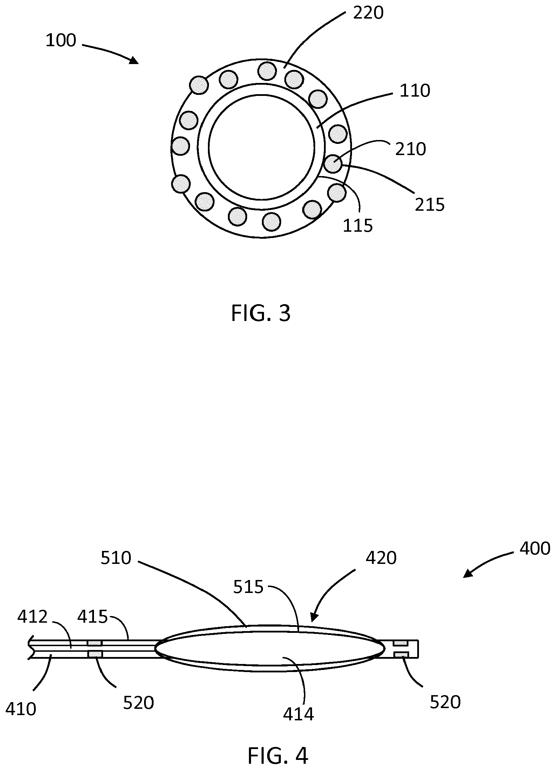

Referring now to **FIG. 3****,** a sectional view of a medical device **100** is shown. The device **100** includes a structural element **110** and coating **220** on an external surface **115** of the structural element. The coating **220** comprises gas-containing material **210** disposed in, for example, microspheres **215.**

It will be understood that **FIGS. 1-3** depict only some ways in which a gas-containing material may be disposed on a structural element of a medical device and that other orientations are envisioned. For example, while microspheres **215** are depicted in **FIG. 3****,** in other embodiments the gas containing material **210** may be entrained within the coating **220** itself, such as by mixing the gas containing material **210** within the coating **220** while the coating is in a liquid state.

In embodiments where the structural element is an inflatable balloon, the balloon may be coated while inflated or deflated and folded. The gas-containing material or the coating material may be placed on an external surface of the balloon by, for example, dipping, spraying, or otherwise depositing a solution containing the material or coating on the balloon and evaporating a solvent, leaving the material or coating deposited on the balloon. If the balloon is folded, the material or coating may penetrate under the folds by capillarity action or may be applied, by for example, micro-nozzles under the folds. One or more layers of a solution comprising the material or coating may be applied to the balloon. Alternatively, a sheet comprising the material or coating may be placed around the balloon.

Referring now to **FIG. 4****,** a sectional view of a balloon catheter **400** is shown. The balloon catheter **400** includes a catheter **410** and a balloon **420,** which are structural elements of the medical device. The catheter **410** defines a lumen **412** in communication with the interior **414** of the balloon **420** for inflating the balloon **420.** The balloon catheter **400** further includes optional rings **520** of gas-containing material embedded in the catheter **410** and exposed on an external surface **415** for contact with physiological fluid when inserted in a patient. Gas-containing material **510** is also disposed on an exterior surface **515** of the balloon **420.** When inserted into a patient, gas bubbles may be released from the material **510** disposed on the balloon **420** and the rings **520** upon contact of the material with physiological fluid. The gas bubbles may enhance ultrasound echogenicity enabling effective ultrasound imaging of the balloon **420** and rings **520** as the balloon catheter **400** is advanced through the patient. Inflation of the balloon **420** may also effectively be imaged by ultrasound via gas released from material **510** on balloon **420.** Further, as the released gas disperses into the vessel, the vessel may become more easily imaged under the ultrasound, further enhancing the ability of the physician to visualize the procedure.

All scientific and technical terms used herein have meanings commonly used in the art unless otherwise specified. The definitions provided herein are to facilitate understanding of certain terms used frequently herein and are not meant to limit the scope of the present disclosure.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the inventive technology.

It will be apparent to those skilled in the art that various modifications and variations can be made to the present inventive technology without departing from the scope of the disclosure.

## Claims

1. A medical device, comprising:
a structural element comprising an outer surface; and
a material on the outer surface of the structural element, where the material dissolves in physiological fluid, the material defining void spaces containing a gas entrained within the void spaces,
wherein, upon contact with physiological fluid, the material is configured to release the gas in an amount sufficient for the released gas to be imaged by extracorporeal ultrasound.

2. The medical device of claim 1, further comprising a coating on the outer surface of the device, wherein the coating comprises the material.

3. The medical device of claim 2, where the coating comprises a hydrogel.

4. The medical device of any one of claims 1-3, wherein the material has a solubility of 100 mg/ml or more in pH 7.4 phosphate buffered saline at room temperature.

5. The medical device of any one of claims 1 or 4, wherein the material defining the void space in which the gas is entrained comprises a monosaccharide or polysaccharide.

6. The medical device of claim 5, wherein the monosaccharide or polysaccharide comprises sucrose or glucose.

7. The medical device of any one of claims 1-6, further comprising an outer coating disposed over the material defining the void space in which the gas is entrained.

8. The medical device of claim 7, wherein the outer coating is a hydrophilic lubricous coating.

9. The medical device of any one of claims 1-8, wherein the material defining the void space in which the gas is entrained comprises a plurality of microspheres, each defining an interior volume, and wherein the gas is present in the interior volume.

10. The medical device of claim 9, wherein a liquid is in the interior volume of the microspheres and wherein the gas is entrained in the liquid.

11. The medical device of any one of claims 1-10, wherein the gas comprises carbon dioxide.

12. The medical device of any one of claims 1-11, wherein the structural element comprises an inflatable balloon, and/or wherein the structural element comprises a catheter.

13. A method of making a medical device, comprising:
entraining a gas into a material, where the material dissolves in physiological fluid, wherein the material defines void spaces and the gas is entrained within the void spaces; and
disposing the material on an outer surface of a structural element of the medical device;
wherein the material is configured to release the gas from the voids in the material upon contact with a physiological fluid in an amount sufficient for the released gas to be imaged by extracorporeal ultrasound.

14. The method of claim 13, wherein the structural element is an inflatable balloon.

15. The method of any one of claims 13 or 14, wherein the gas is carbon dioxide.

16. The method of any one of claims 13-15, wherein the material is selected from the group consisting of a water-soluble polymer, a monosaccharide, a polysaccharide, and microspheres.

## Patentansprüche

1. Medizinische Vorrichtung, welche umfasst:
ein Strukturelement, das eine Außenfläche umfasst; und
ein Material an der Außenfläche des Strukturelements, wobei sich das Material in physiologischem Fluid auflöst, wobei das Material Hohlräume definiert, die ein Gas enthalten, das in den Hohlräumen mitgeführt wird,
wobei das Material dazu konfiguriert ist, das Gas bei Kontakt mit dem physiologischen Fluid in einer Menge freizusetzen, die ausreicht, damit das freigesetzte Gas durch extrakorporalen Ultraschall abgebildet werden kann.

2. Medizinische Vorrichtung nach Anspruch 1, ferner umfassend eine Beschichtung an der Außenfläche der Vorrichtung, wobei die Beschichtung das Material umfasst.

3. Medizinische Vorrichtung nach Anspruch 2, wobei die Beschichtung ein Hydrogel umfasst.

4. Medizinische Vorrichtung nach einem der Ansprüche 1 bis 3, wobei das Material eine Löslichkeit von 100 mg/ml oder mehr in einer phosphatgepufferten Salzlösung mit einem pH von 7,4 bei Raumtemperatur aufweist.

5. Medizinische Vorrichtung nach einem der Ansprüche 1 oder 4, wobei das Material, das den Hohlraum definiert, in dem das Gas mitgeführt wird, ein Monosaccharid oder Polysaccharid umfasst.

6. Medizinische Vorrichtung nach Anspruch 5, wobei das Monosaccharid oder Polysaccharid Saccharose oder Glucose umfasst.

7. Medizinische Vorrichtung nach einem der Ansprüche 1 bis 6, ferner umfassend eine Außenbeschichtung, die über dem Material angeordnet ist, das den Hohlraum definiert, in dem das Gas mitgeführt wird.

8. Medizinische Vorrichtung nach Anspruch 7, wobei die Außenbeschichtung eine hydrophile schmierende Beschichtung ist.

9. Medizinische Vorrichtung nach einem der Ansprüche 1 bis 8, wobei das Material, das den Hohlraum definiert, in dem das Gas mitgeführt wird, eine Vielzahl von Mikrokugeln umfasst, die jeweils ein Innenvolumen definieren, und wobei das Gas in dem Innenvolumen vorliegt.

10. Medizinische Vorrichtung nach Anspruch 9, wobei eine Flüssigkeit im Innenvolumen der Mikrokugeln vorliegt und wobei das Gas in der Flüssigkeit mitgeführt wird.

11. Medizinische Vorrichtung nach einem der Ansprüche 1 bis 10, wobei das Gas Kohlendioxid umfasst.

12. Medizinische Vorrichtung nach einem der Ansprüche 1 bis 11, wobei das Strukturelement einen aufblasbaren Ballon umfasst und/oder wobei das Strukturelement einen Katheter umfasst.

13. Verfahren zum Herstellen einer medizinischen Vorrichtung, umfassend:
Mitführen eines Gases in einem Material, wobei sich das Material in physiologischem Fluid auflöst, wobei das Material Hohlräume definiert und das Gas in den Hohlräumen mitgeführt wird; und
Anordnen des Materials auf einer Außenfläche eines Strukturelements der medizinischen Vorrichtung;
wobei das Material dazu konfiguriert ist, das Gas bei Kontakt mit dem physiologischen Fluid in einer Menge aus den Hohlräumen freizusetzen, die ausreicht, damit das freigesetzte Gas durch extrakorporalen Ultraschall abgebildet werden kann.

14. Verfahren nach Anspruch 13, wobei das Strukturelement ein aufblasbarer Ballon ist.

15. Verfahren nach einem der Ansprüche 13 oder 14, wobei das Gas Kohlendioxid ist.

16. Verfahren nach einem der Ansprüche 13 bis 15, wobei das Material ausgewählt wird aus der Gruppe bestehend aus wasserlöslichem Polymer, einem Monosaccharid, einem Polysaccharid und Mikrokugeln.

## Revendications

1. Dispositif médical, comprenant :
un élément structural comprenant une surface extérieure ; et
un matériau sur la surface extérieure de l'élément structural, où le matériau se dissout dans un fluide physiologique, le matériau définissant des espaces vides contenant un gaz entraîné à l'intérieur des espaces vides,
dans lequel, lors du contact avec le fluide physiologique, le matériau est configuré pour libérer le gaz en une quantité suffisante pour que le gaz libéré apparaisse sur une imagerie par ultrasons extracorporels.

2. Dispositif médical selon la revendication 1, comprenant en outre un revêtement sur la surface extérieure du dispositif, dans lequel le revêtement comprend le matériau.

3. Dispositif médical selon la revendication 2, dans lequel le revêtement comprend un hydrogel.

4. Dispositif médical selon l'une quelconque des revendications 1 à 3, dans lequel le matériau a une solubilité de 100 mg/ml ou plus dans une solution saline tamponnée de phosphate avec un pH de 7,4 à température ambiante.

5. Dispositif médical selon l'une quelconque des revendications 1 ou 4, dans lequel le matériau définissant l'espace vide dans lequel le gaz est entraîné comprend un monosaccharide ou un polysaccharide.

6. Dispositif médical selon la revendication 5, dans lequel le monosaccharide ou le polysaccharide comprend du saccharose ou du glucose.

7. Dispositif médical selon l'une quelconque des revendications 1 à 6, comprenant en outre un revêtement extérieur disposé sur le matériau définissant l'espace vide dans lequel le gaz est entraîné.

8. Dispositif médical selon la revendication 7, dans lequel le revêtement extérieur est un revêtement lubrifiant hydrophile.

9. Dispositif médical selon l'une quelconque des revendications 1 à 8, dans lequel le matériau définissant l'espace vide dans lequel le gaz est entraîné comprend une pluralité de microsphères, chacune définissant un volume intérieur, et dans lequel le gaz est présent dans le volume intérieur.

10. Dispositif médical selon la revendication 9, dans lequel un liquide se trouve dans le volume intérieur des microsphères et dans lequel le gaz est entraîné dans le liquide.

11. Dispositif médical selon l'une quelconque des revendications 1 à 10, dans lequel le gaz comprend du dioxyde de carbone.

12. Dispositif médical selon l'une quelconque des revendications 1 à 11, dans lequel l'élément structural comprend un ballonnet gonflable, et/ou dans lequel l'élément structural comprend un cathéter.

13. Procédé de fabrication d'un dispositif médical, comprenant :
l'entraînement d'un gaz dans un matériau, où le matériau se dissout dans un fluide physiologique, dans lequel le matériau définit des espaces vides et le gaz est entraîné à l'intérieur des espaces vides ; et
la disposition du matériau sur une surface extérieure d'un élément structural du dispositif médical ;
dans lequel le matériau est configuré pour libérer le gaz des vides dans le matériau lors du contact avec un fluide physiologique en une quantité suffisante pour que le gaz libéré apparaisse sur une imagerie par ultrasons extracorporels.

14. Procédé selon la revendication 13, dans lequel l'élément structural est un ballonnet gonflable.

15. Procédé selon l'une quelconque des revendications 13 ou 14, dans lequel le gaz est du dioxyde de carbone.

16. Procédé selon l'une quelconque des revendications 13 à 15, dans lequel le matériau est sélectionné dans le groupe constitué d'un polymère hydrosoluble, d'un monosaccharide, d'un polysaccharide et de microsphères.
